# FASCICULE DE BREVET EUROPEEN

(11) **EP 1 245 568 B1**
(45) Date de publication et mention de la délivrance du brevet: **18.01.2006**
(21) Numéro de dépôt: 02290755.4
(22) Date de dépôt: 27.03.2002
(51) Int. Cl.: C07D 295/18, A61K 31/395, A61P 43/00, C07D 277/10, C07K 5/06, C07D 207/16, C07D 231/04, C07D 277/04, C07D 277/06, C07D 233/06

(54) **Dérives sulfonyles d'-aminoacides et leur utilisation en tant qu'inhibiteurs de dipeptidyl-peptidase IV ( DPP IV)**
Sulfonylderivate von Aminosäuren und deren Verwendung als Inhibitoren der Dipeptidyl-peptidase IV (DPP IV)
Sulfonyl derivatives of amino acids and their use as inhibitors of dipeptidyl-peptidase IV (DPP IV)

(30) Priorité: 28.03.2001 FR 0104142
(43) Date de publication de la demande: 02.10.2002
(73) Titulaire: Les Laboratoires Servier, 92200 Neuilly-sur-Seine (FR)
(72) Inventeur: De Nanteuil, Guillaume, 92150 Suresnes (FR); Portevin, Bernard, 78990 Elancourt (FR); Benoist, Alain, 95130 Franconville (FR); Levens, Nigel, 92420 Vaucresson (FR); Nosjean, Olivier, 92500 Rueil Malmaison (FR); Husson-Robert, Bernadette, 92000 Nanterre (FR)

(56) Documents cités:
- WO-A-95/15309
- US-A- 6 110 949

## Description

La présente invention concerne de nouveaux dérivés sulfonylés d'α-amino-acides, leur procédé de préparation, les compositions pharmaceutiques qui les contiennent ainsi que leur utilisation en tant qu'inhibiteurs de dipeptidyl-peptidase IV (DPP IV).

Des inhibiteurs de DPP IV ont déjà été décrits dans la littérature, notamment des dérivés d'amides dans la demande de brevet EP 0 490 379 et dans le journal Adv. Exp. Med. Biol. 1997, 421, 157-160, des dérivés de carbamates dans la demande de brevet DE 19826972, des dérivés de N-glycyl-4-cyanothiazolidines dans le brevet US 6110949 et des dérivés de sulfones dans les demandes de brevets WO 95/15309 et WO 01/81304.

La dipeptidyl-peptidase IV est une sérine protéase membranaire, présente dans de nombreux tissus humains, et impliquée dans de nombreuses pathologies :
- Il a été montré que la DPP IV était responsable de l'inactivation du GLP-1 (glucagon-like peptide-1). Or, le GLP-1 est un important stimulateur de la sécrétion d'insuline dans le pancréas et a donc un effet bénéfique direct sur le taux de glucose dans le sang. L'inhibition de la DPP IV représente donc une approche extrêmement intéressante dans le traitement de l'intolérance au glucose et des maladies associées à l'hyperglycémie, telles que, par exemple, le diabète non insulino-dépendant (diabète de type II) ou l'obésité.
- Il a également été montré que la DPP IV jouait un rôle dans la réponse immunitaire. Exprimée par les lymphocytes T-CD₄₊ où elle est synonyme de l'antigène CD26, elle joue un rôle important dans le mécanisme du rejet de greffe (Transplantation 1997, 63 (10), 1495-500).
   En permettant une suppression plus sélective de la réponse immunitaire, l'inhibition de la DPP IV représente donc une approche extrêmement intéressante dans la prévention du rejet de greffe chez les transplantés.
- Il a également été montré que la DPP IV endothéliale du poumon, en se liant à la fibronectine des cellules cancéreuses, en favorisait la métastase (J. Biol. Chem. 1998, 273 (37), 24207-24215).
   Des inhibiteurs de DPP IV sont donc utiles dans le traitement du cancer et la prévention des métastases cancéreuses.
- La DPP IV joue également un rôle important dans l'infection des lymphocytes T-CD₄₊ par le virus HIV-1 (Res. Virol. 1997, 148 (4), 255-266). En empêchant la pénétration du virus dans les cellules, les inhibiteurs de DPP IV sont donc particulièrement intéressants dans la prévention de la transmission du virus HIV-1. Il a aussi été montré que la DPP IV était responsable de l'inactivation de chimiokines telles que les facteurs SDF-1α et SDF-1β, connus pour leur activité chimiotactique et antivirale (Proc. Natl. Acad. Sci. USA 1998, 95 (11), 6331-6336).
- La DPP IV jouerait également un rôle important dans la pathogenèse de la parodontite (Infect. Immun. 2000, 68 (2), 716-724).
- Enfin, il a été montré que la DPP IV était responsable de l'inactivation du GLP-2, facteur facilitant l'adaptation de l'intestin après une résection importante (J. Surg. Res. 1999, 87 (1), 130-133).
   Des inhibiteurs de DPP IV sont donc potentiellement utiles dans l'adaptation intestinale.

Plus spécifiquement, la présente invention concerne les composés de formule (I) : 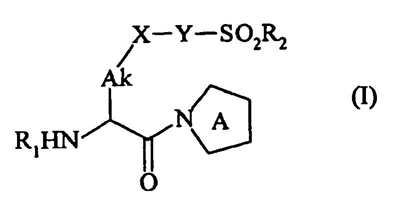
dans laquelle :
- 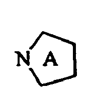 représente un hétérocycle azoté à 5 chaînons éventuellement substitué par un groupement cyano,
- R₁ représente un atome d'hydrogène ou un groupement alkyle (C₁-C₆) linéaire ou ramifié, acyle (C₁-C₆) linéaire ou ramifié, prolyle, alanyle, histidylprolyle ou phénylalanylprolyle,
- Ak représente une chaîne alkylène (C₁-C₆) linéaire ou ramifiée ou hétéroalkylène,
- X représente une liaison simple ou un groupement phénylène éventuellement substitué par un ou plusieurs groupements, identiques ou différents, choisis parmi les atomes d'halogène et les groupements alkyle (C₁-C₆) linéaire ou ramifié, hydroxy, alkoxy (C₁-C₆) linéaire ou ramifié, ou polyhalogénoalkyle (C₁-C₆) linéaire ou ramifié,
- R₂ représente un groupement cycloalkyle (C₃-C₁₀),
- Y représente un groupement NH,
leurs isomères optiques et leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique, etc.

Par hétérocycle azoté à 5 chaînons, on entend un groupement monocyclique saturé de 5 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.
Les hétérocycles azotés préférés sont les groupements pyrrolidinyle, thiazolidinyle, isoxazolidinyle, et pyrazolidinyle.

Par chaîne hétéroalkylène, on entend une chaîne alkylène (C₁-C₆) linéaire ou ramifiée dans laquelle un chaînon -CH₂- a été remplacé par un atome d'oxygène ou de soufre.

Les composés préférés de formule (I) sont ceux pour lesquels 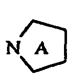 représente un groupement 1-pyrrolidinyle éventuellement substitué par un groupement cyano, ou 1,3-thiazolidin-3-yle éventuellement substitué par un groupement cyano.

Les composés préférés de formule (I) sont ceux pour lesquels X représente une liaison simple.

Les composés préférés de formule (I) sont ceux pour lesquels la configuration en α de la fonction amide 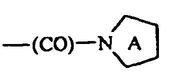 est (S).

Parmi les composés préférés de l'invention, on peut citer plus particulièrement :
le (S)-N-[4-amino-5-oxo-5-(1-pyrrolidinyl)-pentyl]-cyclohexanesulfonamide, ses isomères optiques ainsi que ses sels d'addition à un acide pharmaceutiquement acceptable.

L'invention s'étend également au procédé de préparation des composés de formule (I), caractérisé en ce que l'on met en réaction un composé de formule (II) : 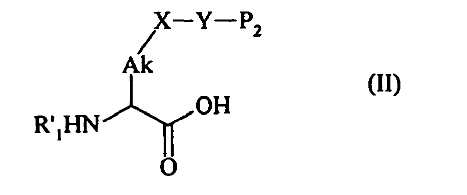 dans laquelle Ak, X et Y sont tels que définis dans la formule (I), R'₁ représente un groupement protecteur de la fonction amino ou un groupement alkyle (C₁-C₆) linéaire ou ramifié, acyle (C₁-C₆) linéaire ou ramifié, prolyle éventuellement protégé par un groupement protecteur de la fonction amino, alanyle éventuellement protégé par un groupement protecteur de la fonction amino, histidylprolyle éventuellement protégé par un groupement protecteur de la fonction amino ou phénylalanylprolyle éventuellement protégé par un groupement protecteur de la fonction amino, et P₂ représente un atome d'hydrogène ou un groupement protecteur de la fonction amino, avec un composé de formule (III) : 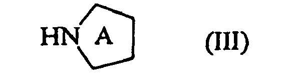
dans laquelle 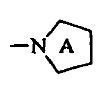 est tel que défini dans la formule (I), dans des conditions classiques de couplage peptidique,
pour conduire après déprotection éventuelle du groupement Y au composé de formule (IV) : 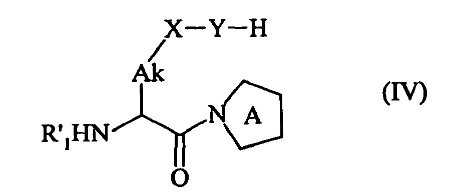 dans laquelle 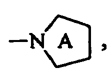 Ak, X, Y et R'₁ ont la même signification que précédemment, que l'on met ensuite en réaction avec un composé de formule (V) :

R₂-SO₂-Z₁ (V)

dans laquelle R₂ est tel que défini dans la formule (I), et Z₁ représente un groupement partant tel que, par exemple, un atome d'halogène,
pour conduire après déprotection éventuelle au composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

Les composés de la présente invention, outre le fait qu'ils soient nouveaux, présentent des propriétés pharmacologiques intéressantes. Ils ont des propriétés inhibitrices de la dipeptidyl-peptidase IV qui les rendent utiles dans le traitement de l'intolérance au glucose, des maladies associées à une hyperglycémie telles que le diabète de type II ou l'obésité, dans la prévention du rejet de greffe après transplantation, dans le traitement du cancer et la prévention des métastases cancéreuses, dans le traitement du sida, de la parodontite, ainsi que dans l'adaptation intestinale après résection.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention.
Les composés A et B sont des composés n'appartenant pas à l'invention.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

### Composé A : (S)-1-[N⁵-{(Imino)-(méthylsulfonylamino)-méthyl}-ornithyl]-pyrrolidine, dichlorhydrate

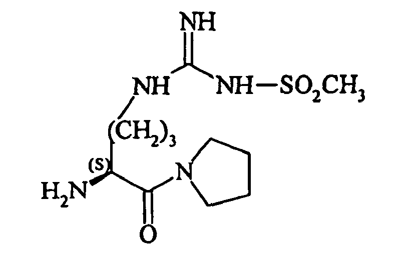

### Stade A : (S)-N²-(Benzyloxycarbonyl)-N⁵-{(imino)-(méthylsulfonylamino)-méthyl}-ornithine

A 10 mmoles de (S)-N²-(benzyloxycarbonyl)-arginine en suspension dans un mélange acétone/eau 80/20 est ajoutée, à 0°C, une solution 4N de soude, en quantité suffisante pour amener le pH du milieu réactionnel entre 11 et 11,5. Après 2 heures d'agitation est ensuite ajoutée une solution de chlorure de méthanesulfonyle dans l'acétone, puis, après 3 heures d'agitation supplémentaires, le milieu est neutralisé à l'aide d'une solution 1N d'acide chlorhydrique, puis l'acétone est évaporée, le résidu obtenu est extrait à l'éther puis lavé, séché, filtré et évaporé. L'huile jaune pâle ainsi obtenue est purifiée par chromatographie sur silice (éluant : dichlorométhane/méthanol 7/3) pour conduire au produit attendu sous la forme d'une meringue blanchâtre.

### Stade B : (S)-1-[N²-(Benzyloxycarbonyl)-N⁵-{(imino)-(méthylsulfonylamino)-méthyl}-ornithyl]-pyrrolidine

A 10 mmoles du composé obtenu dans le stade précédent en solution dans la diméthylformamide sont ajoutées, à 0°C, 10 mmoles de N-méthyl-morpholine, puis, en 10 minutes, 10 mmoles de chloroformiate d'isobutyle, enfin, en 10 minutes, 10 mmoles de pyrrolidine en solution dans la diméthylformamide. Après une nuit d'agitation à température ambiante, la diméthylformamide est évaporée, puis une solution d'hydrogénocarbonate de sodium est ajoutée au résidu obtenu, qui est alors extrait au dichlorométhane. Les phases organiques rassemblées sont ensuite lavées, d'abord avec une solution d'acide citrique à 10 %, puis à l'eau, puis elles sont séchées, filtrées et évaporées. L'huile jaune ainsi obtenue est purifiée par chromatographie sur silice (éluant : dichlorométhane/méthanol 95/5) pour conduire au produit attendu sous la forme d'une meringue blanchâtre.

### Stade C : (S)-1-[N⁵-{(Imino)-(méthylsulfonylamino)-méthyl}-ornithyl]-pyrrolidine

10 mmoles du composé obtenu dans le stade précédent en solution dans l'éthanol sont hydrogénées en présence de palladium sur charbon à 10 %, à température et pression ambiantes, pendant 6 heures. Le milieu réactionnel est ensuite filtré et évaporé, puis, après addition d'eau, il est lyophilisé pour conduire au produit attendu.

**Microanalyse élémentaire :**

| | C% | *H%* | *N%* | S% |
|---|---|---|---|---|
| *calculé* | *43,26* | *7,59* | *22,93* | *10,50* |
| *trouvé* | *43, 45* | *7, 62* | *22, 32* | *10,33* |

### Stade D : (S)-1-[N⁵-{(Imino)-(méthylsulfonylamino)-méthyl}-ornithyl]-pyrrolidine, dichlorhydrate

A 10 mmoles du composé obtenu dans le stade précédent en solution dans le dioxanne est ajoutée une solution 4N d'acide chlorhydrique dans le dioxanne. Après 24 heures d'agitation à température ambiante, le solvant est évaporé, de l'eau est ajoutée et la solution lyophilisée pour conduire au produit attendu.

**Microanalyse élémentaire :**

| | *C%* | *H%* | *N%* | *S%* | *Cl %* |
|---|---|---|---|---|---|
| *calculé* | *34,92* | *6,67* | *18,52* | *8,47* | *18,74* |
| *trouvé* | *34,42* | *6, 60* | *17,78* | *8, 46* | *19,52* |

### Composé B : (S)-N-[4-Amino-5-oxo-5-(1-pyrrolidinyl)-pentyl]-méthane-sulfonamide, chlorhydrate

### Stade A : (S)-N¹-(Benzyloxycarbonyl)-N⁴-(tert-butyloxycarbonyl)-5-oxo-5-(1-pyrrolidinyl)-1,4-pentanediamine

A 10 mmoles de (S)-N⁵-(benzyloxycarbonyl)-N²-(tert-butyloxycarbonyl)-ornithine en solution dans la diméthylformamide sont ajoutées 10 mmoles de pyrrolidine, 10 mmoles de 1-hydroxybenzotriazole et 10 mmoles de dicyclohexylcarbodiimide. Après une nuit d'agitation à température ambiante, la dicyclohexylurée formée est filtrée, puis la diméthylformamide évaporée. L'huile marron ainsi obtenue est purifiée par chromatographie sur silice (éluant : dichlorométhane/éthanol 95/5) pour conduire au produit attendu sous la forme d'une huile jaune.

### Stade B : (S)-N⁴-(Tert-butyloxycarbonyl)-5-oxo-5-(1-pyrrolidinyl)-1,4-pentanediamine

Le produit attendu est obtenu selon le procédé décrit dans le stade C du composé A, à partir du composé obtenu dans le stade précédent.

### Stade C : (S)-N-[4-(Tert-butyloxycarbonylamino)-5-oxo-5-(1-pyrrolidinyl)-pentyl]-méthanesulfonamide

A 10 mmoles du composé obtenu dans le stade précédent en solution dans la pyridine sont ajoutées 10 mmoles de chlorure de méthanesulfonyle en solution dans le dichlorométhane. Après une nuit d'agitation à température ambiante, les solvants sont évaporés. Au résidu obtenu est ensuite ajouté de l'acétate d'éthyle, puis la solution est lavée, séchée, filtrée et évaporée. L'huile obtenue est purifiée par chromatographie sur silice (éluant : dichlorométhane/méthanol 95/5) pour conduire au produit attendu sous la forme d'une huile.

### Stade D: (S)-N-[4-Amino-5-oxo-5-(1-pyrrolidinyl)-pentyl]-méthanesulfonamide, chlorhydrate

Le produit attendu est obtenu selon le procédé décrit dans le stade D du composé A à partir du composé obtenu au stade précédent.

**Microanalyse élémentaire :**

| | *C%* | *H%* | *N%* | *S%* | *Cl %* |
|---|---|---|---|---|---|
| *calculé* | *40,06* | *7,40* | *14,02* | *10,69* | *11,82* |
| *trouvé* | *39,99* | *7,75* | *13,95* | *9,81* | *13,15* |

### EXEMPLE 29 : (S)-N-[4-Amino-5-oxo-5-(1-pyrrolidinyl)-pentyl]-cyclopropane-sulfonamide, chlorhydrate

Le produit attendu est obtenu selon le procédé décrit pour le composé B à partir de (S)-N⁵-(benzyloxycarbonyl)-N²-(tert-butyloxycarbonyl)-ornithine, de chlorure de cyclopropane-sulfonyle et de pyrrolidine.

**Microanalyse élémentaire :**

| | *C%* | *H%* | *N%* | *S%* | *Cl %* |
|---|---|---|---|---|---|
| *calculé* | *44,23* | *7,42* | *12,90* | *9,84* | *10,88* |
| *trouvé* | *44, 06* | *7, 83* | *12,62* | *10, 06* | *10, 67* |

### EXEMPLE 31 : (S)-N-[4-Amino-5-oaco-5-(1-pyrrolidinyl)-pentyl]-cyclobutane-sulfonamide, chlorhydrate

Le produit attendu est obtenu selon le procédé décrit pour le composé B à partir de (S)-N⁵⁻(benzyloxycarbonyl)-N²-(tert-butyloxycarbonyl)-ornithine, de chlorure de cyclobutane-sulfonyle et de pyrrolidine.

**Microanalyse élémentaire :**

| | *C%* | *H%* | *N%* | *S%* | *Cl%* |
|---|---|---|---|---|---|
| *calculé* | *45,94* | *7,71* | *12,36* | *9,43* | *10,43* |
| *trouvé* | *47,08* | *7,82* | *11,95* | *9,29* | *10,73* |

### EXEMPLE 33 : (S)-N-[4-Amino-5-oxo-5-(1-pyrrolidinyl)-pentyl)-cyclopentane-sulfonamide, chlorhydrate

Le produit attendu est obtenu selon le procédé décrit pour le composé B à partir de (S)-N⁵⁻(benzyloxycarbonyl)-N²-(tert-butyloxycarbonyl)-ornithine, de chlorure de cyclopentane-sulfonyle et de pyrrolidine.

**Microanalyse élémentaire :**

| | C% | *H%* | *N%* | S% | *Cl%* |
|---|---|---|---|---|---|
| *calculé* | *47,51* | *7,97* | *11,87* | *9,06* | *10,02* |
| *trouvé* | *47, 08* | *7,98* | *11,41* | *8,95* | *9,96* |

### EXEMPLE 35 : (S)-N-[4-Amino-5-oxo-5-(1-pyrrolidinyl)-pentyl]-cyclohexane-sulfonamide, chlorhydrate

Le produit attendu est obtenu selon le procédé décrit pour le composé B à partir de (S)-N⁵⁻(benzyloxycarbonyl)-N²-(tert-butyloxycarbonyl)-ornithine, de chlorure de cyclohexanesulfonyle et de pyrrolidine.

**Microanalyse élémentaire :**

| | C% | *H%* | *N%.* | S% | *Cl%* |
|---|---|---|---|---|---|
| *calculé* | *48,97* | *8,22* | *11,42* | *8,71* | *9,64* |
| *trouvé* | *48, 68* | *8, 26* | *11,20* | *8, 34* | *9, 44* |

### EXEMPLE 59 : (S)-N-[5-Amino-6-oxo-6-(1,3-thiazolidin-3-yl)-hexyl]-cyclohexane-sulfonamide, chlorhydrate.

Le produit attendu est obtenu selon le procédé décrit pour le composé B à partir de (S)-N⁶-(benzyloxycarbonyl)-N²-(tert-butyloxycarbonyl)-lysine, de chlorure de cyclohexanesulfonyle et de 1,3-thiazolidine.

**Microanalyse élémentaire :**

| | *C%* | *H%* | *N%* | *S%* | *Cl %* |
|---|---|---|---|---|---|
| *calculé* | *45,04* | *7,56* | *10,50* | *16,03* | *8,86* |
| *trouvé* | *45,36* | *7,37* | *10,22* | *16,69* | *9,39* |

### EXEMPLE 62 : N-{(5S)-5-Amino-6-oxo-6-[(4R)-4-cyano-1,3-tbiazolidin-3-yl]-heayl}-cyclohexanesulfonamide, chlorhydrate

Le produit attendu est obtenu selon le procédé décrit pour le composé B, à partir de (S)-N⁶⁻(benzyloxycarbonyl)-N²-(tert-butyloxycarbonyl)-lysine, de chlorure de cyclohexanesulfonyle et de (R)-4-cyano-1,3-thiazolidine.

**Microanalyse élémentaire :**

| | *C%* | *H%* | *N%* | *S%* | *Cl %* |
|---|---|---|---|---|---|
| *calculé* | *45,22* | *6,88* | *13,18* | *15,09* | *8,34* |
| *trouvé* | *45,41* | *6, 75* | *12,94* | *15,09* | *8,75* |

### EXEMPLE 64 : N-[(5S)-5-Amino-6-oxo-6-{(2S)-2-cyano-1-pyrrolidinyl}-hexyl]-cyclohexanesulfonamide, chlorhydrate

Le produit attendu est obtenu selon le procédé décrit pour le composé B, à partir de (S)-N⁶-(benzyloxycarbonyl)-N²-(tert-butyloxycarbonyl)-lysine, de chlorure de cyclohexanesulfonyle et de chlorydrate de (S)-2-cyano-pyrrolidine.

**Microanalyse élémentaire :**

| | *C%* | *H%* | *N%* | *S%* | *Cl %* |
|---|---|---|---|---|---|
| *calculé* | *50,17* | *7,68* | *13,77* | *7,88* | *8,71* |
| *trouvé* | *50,25* | *7,71* | *14,07* | *7,71* | *8,31* |

### EXEMPLE 65 : (S)-N-[5-Amino-6-oxo-6-(1-pyrrolidinyl)-hexyl]-cyclohexanesulfonamide, chlorhydrate

Le produit attendu est obtenu selon le procédé décrit pour le composé B à partir de (S)-N⁶-(benzyloxycarbonyl)-N²-(tert-butyloxycarbonyl)-lysine, de chlorure de cyclohexanesulfonyle et de pyrrolidine.

**Microanalyse élémentaire :**

| | *C%* | *H%* | *N%* | *S%* | *Cl %* |
|---|---|---|---|---|---|
| *calculé* | *50,31* | *8,44* | *11,00* | *8,39* | 9,28 |
| *trouvé* | *50,44* | *8,44* | *10,93* | *7,99* | *9,31* |

### EXEMPLE 66 : (S)-N-[4-Amino-5-oxo-5-(1,3-thiazolidin-3-yl)-pentyl]-cyclobexanesulfonamide, chlorhydrate

Le produit attendu est obtenu selon le procédé décrit pour le composé B, en remplaçant la pyrrolidine par la 1,3-thiazolidine, et le chlorure de méthanesulfonyle par le chlorure de cyclohexanesulfonyle.

**Microanalyse élémentaire :**

| | *C%* | *H%* | *N%* | S% | *Cl%* |
|---|---|---|---|---|---|
| *calculé* | *43,57* | *7,31* | *10,89* | *16,62* | *9,19* |
| *trouvé* | *43,40* | *7,30* | *10,52* | *16,64* | *9,34* |

### EXEMPLE 74 N-[(4S)-4-Amino-5-oxo-5-((2S)-2-cyano-1-pyrrolidinyl)-pentyl]-cyclohexanesulfonamide, chlorhydrate

Le produit attendu est obtenu selon le procédé décrit pour le composé B à partir de (S)-N⁵-(benzyloxycarbonyl)-N²-(tert-butyloxycarbonyl)-ornithine, de chlorure de cyclohexanesulfonyle et de chlorhydrate de (S)-2-cyano-pyrrolidine.

### ETUDE PHARMACOLOGIQUE DES COMPOSES DE L'INVENTION

### EXEMPLE 77: Inhibition de la dipeptidyl-peptidase IV in vitro

L'effet des composés sur l'activité enzymatique *in vitro* de la DPPIV est évalué comme suit. L'enzyme de rein de porc est dosée à l'aide d'un substrat chromogène, le glycyl-prolyl-*p*-nitroanilide 1,4 mM, qui est hydrolysé pour libérer de lap-nitroaniline absorbant à 405 nm. L'activité de l'enzyme est déterminée par absorbance, en présence de concentrations variables du composé à évaluer (10⁻⁴ à 10⁻⁷ M le plus souvent). Les données obtenues permettent de déterminer la dose efficace pour l'inhibition à 50 % de l'activité contrôle (IC₅₀). Les composés de l'invention possèdent une IC₅₀ comprise entre 1nM et 20 µM.

### EXEMPLE 78 : Composition pharmaceutique

Formule de préparation pour 1000 comprimés dosés à 10 mg

| | |
|---|---|
| Composé A | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 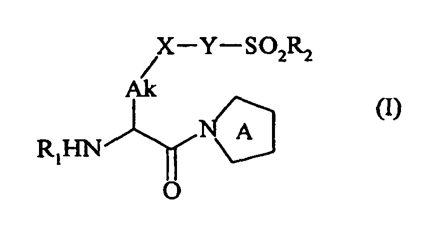
dans laquelle :
- 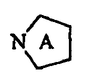 représente un hétérocycle azoté à 5 chaînons éventuellement substitué par un groupement cyano,
- R₁ représente un atome d'hydrogène ou un groupement alkyle (C₁-C₆) linéaire ou ramifié, acyle (C₁-C₆) linéaire ou ramifié, prolyle, alanyle, histidylprolyle ou phénylalanylprolyle,
- Ak représente une chaîne alkylène (C₁-C₆) linéaire ou ramifiée ou hétéroalkylène,
- X représente une liaison simple ou un groupement phénylène éventuellement substitué par un ou plusieurs groupements, identiques ou différents, choisis parmi les atomes d'halogène et les groupements alkyle (C₁-C₆) linéaire ou ramifié, hydroxy, alkoxy (C₁-C₆) linéaire ou ramifié, ou polyhalogénoalkyle (C₁-C₆) linéaire ou ramifié,
- R₂ représente un groupement cycloalkyle (C₃-C₁₀),
- Y représente un groupement NH,
ses isomères optiques et ses sels d'addition à un acide pharmaceutiquement acceptable, étant entendu que par hétérocycle azoté à 5 chaînons, on entend un groupement monocyclique saturé de 5 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre,
et par chaîne hétéroalkylène, on entend une chaîne alkylène (C₁-C₆) linéaire ou ramifiée dans laquelle un chaînon -CH₂- a été remplacé par un atome d'oxygène ou de soufre.

2. Composé de formule (I) selon la revendication 1 tel que 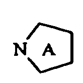 représente un groupement 1-pyrrolidinyle éventuellement substitué par un groupement cyano ou 1,3-thiazolidin-3-yle éventuellement substitué par un groupement cyano.

3. Composé de formule (I) selon la revendication 1 ou 2 tel que X représente une liaison simple.

4. Composé de formule (I) selon l'une quelconque des revendications 1 à 3 tel que la configuration en α de la fonction amide 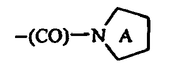 est (S).

5. Composé de formule (I) selon la revendication 1 qui est le (S)-N-[4-amino-5-oxo-5-(1-pyrrolidinyl)-pentyl]-cyclohexanesulfonamide, ses isomères optiques ainsi que ses sels d'addition à un acide pharmaceutiquement acceptable.

6. Procédé de préparation des composés de formule (I) selon la revendication 1 **caractérisé en ce que** l'on met en réaction un composé de formule (II) : 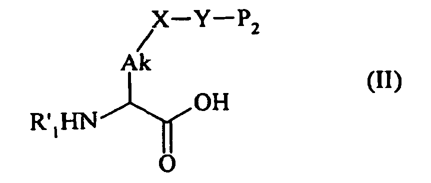 dans laquelle Ak, X et Y sont tels que définis dans la formule (I), R'₁ représente un groupement protecteur de la fonction amino ou un groupement alkyle (C₁-C₆) linéaire ou ramifié, acyle (C₁-C₆) linéaire ou ramifié, prolyle éventuellement protégé par un groupement protecteur de la fonction amino, alanyle éventuellement protégé par un groupement protecteur de la fonction amino, histidylprolyle éventuellement protégé par un groupement protecteur de la fonction amino ou phénylalanylprolyle éventuellement protégé par un groupement protecteur de la fonction amino, et P₂ représente un atome d'hydrogène ou un groupement protecteur de la fonction amino,
avec un composé de formule (III) : 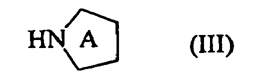 dans laquelle 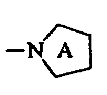 est tel que défini dans la formule (I),
dans des conditions classiques de couplage peptidique,
pour conduire après déprotection éventuelle du groupement Y au composé de formule (IV) : 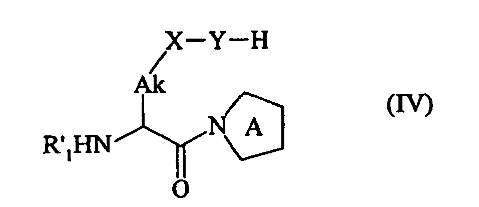 dans laquelle 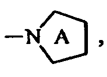 Ak, X, Y et R'₁ ont la même signification que précédemment, que l'on met ensuite en réaction avec un composé de formule (V):
R₂-SO₂-Z₁ (V)
dans laquelle R₂ est tel que défini dans la formule (I), et Z₁ représente un groupement partant tel que, par exemple, un atome d'halogène,
pour conduire après déprotection éventuelle au composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

7. Composition pharmaceutique contenant comme principe actif un composé selon l'une quelconque des revendications 1 à 5, en combinaison avec un ou plusieurs véhicules inertes, non toxiques et pharmaceutiquement acceptables.

8. Composition pharmaceutique selon la revendication 7 utile en tant que médicament comme inhibiteur de dipeptidyl-peptidase IV, utile dans le traitement de l'intolérance au glucose, des maladies associées à une hyperglycémie telles que le diabète de type II ou l'obésité, dans la prévention du rejet de greffe après transplantation, dans le traitement du cancer et la prévention des métastases cancéreuses, dans le traitement du sida, de la parodontite, ainsi que dans l'adaptation intestinale après résection.

9. Composition pharmaceutique selon la revendication 7 utile en tant que médicament antidiabétique.

## Patentansprüche

1. Verbindung der Formel (I): 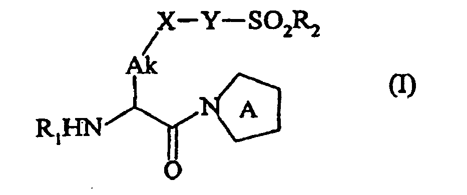 in der:
- 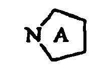 einen Stickstoff-haltigen Heterocyclus mit 5 Kettengliedern, der gegebenenfalls durch eine Cyanogruppe substituiert ist, bedeutet,
- R₁ ein Wasserstoffatom oder eine geradkettige oder verzweigte (C₁-C₆)-Alkylgruppe, geradkettige oder verzweigte (C₁-C₆)-Acylgruppe, Prolylgruppe, Alanylgruppe, Histidylpropylgruppe oder Phenylalanylprolylgruppe bedeutet,
- Ak eine geradkettige oder verzweigte (C₁-C₆)-Alkylen- oder -Heteroalkylenkette bedeutet,
- X eine Einfachbindung oder eine Phenylengruppe, die gegebenenfalls durch eine oder mehrere gleichartige oder verschiedenartige Gruppen ausgewählt aus Halogenatomen, geradkettigen oder verzweigten (C₁-C₆)-Alkylgruppen, Hydroxygruppen, geradkettigen oder verzweigten (C₁-C₆)-Alkoxygruppen oder geradkettigen oder verzweigten (C₁-C₆)-Polyhalogenalkylgruppen substituiert ist,
- R₂ eine (C₃-C₁₀)-Cycloalkylgruppe bedeutet,
- Y eine Gruppe NH bedeutet,
deren optische Isomere und deren Additionssalze mit einer pharmazeutisch annehmbaren Säure,
mit der Maßgabe, daß man unter einem Stickstoff-haltigen Heterocyclus mit 5 Kettengliedern eine gesättigte monocyclische Gruppe mit 5 Kettengliedern versteht, die eines, zwei oder drei Heteroatome aufweisen, wobei eines dieser Heteroatome ein Stickstoffatom ist, und das oder die gegebenenfalls vorhandenen weiteren Heteroatome ausgewählt sind aus Sauerstoff-, Stickstoff- und Schwefelatomen,
und man unter einer Heteroalkylenkette eine geradkettige oder verzweigte (C₁-C₆)-Alkylenkette versteht, bei der ein Kettenglied -CH₂- durch ein Sauerstoffatom oder ein Schwefelatom ersetzt ist.

2. Verbindung der Formel (I) nach Anspruch 1, worin 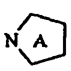 eine 1-Pyrrolidinylgruppe, die gegebenenfalls durch eine Cyanogruppe substituiert ist, oder eine 1,3-Thiazolidin-3-yl-gruppe, die gegebenenfalls durch eine Cyanogruppe substituiert ist, bedeutet.

3. Verbindung der Formel (I) nach Anspruch 1 oder 2, worin X eine Einfachbindung bedeutet.

4. Verbindung der Formel (I) nach einem der Ansprüche 1 bis 3, worin die Konfiguration in α-Stellung zu der Amidfunktion 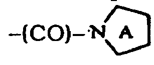 die (S)-Konfiguration ist.

5. Verbindung der Formel (I) nach Anspruch 1, nämlich (S)-N-[4-Amino-5-oxo-5-(1-pyrrolidinyl)-pentyl]-cyclohexansulfonamid, dessen optische Isomeren sowie dessen Additionssalze mit einer pharmazeutisch annehmbaren Säure.

6. Verfahren zur Herstellung der Verbindungen der Formel (I) nach Anspruch 1, **dadurch gekennzeichnet, daß** man eine Verbindung der Formel (II): 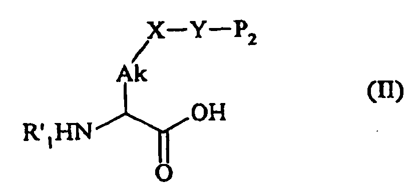 in der Ak, X und Y die bezüglich der Formel (I) angegebenen Bedeutungen besitzen, R'₁ eine Schutzgruppe für die Aminofunktion oder eine geradkettige oder verzweigte (C₁-C₆)-Alkylgruppe, geradkettige oder verzweigte (C₁-C₆)-Acylgruppe, Prolylgruppe, die gegebenenfalls durch eine Schutzgruppe für die Aminofunktion geschützt ist, Alanylgruppe, die gegebenenfalls durch eine Schutzgruppe für die Aminofunktion geschützt ist, Histidylprolylgruppe, die gegebenenfalls durch eine Schutzgruppe für die Aminofunktion geschützt ist, oder Phenylalanylprolylgruppe, die gegebenenfalls durch eine Schutzgruppe für die Aminofunktion geschützt ist, und P₂ ein Wasserstoffatom oder eine Schutzgruppe für die Aminofunktion bedeuten,
mit einer Verbindung der Formel (III): 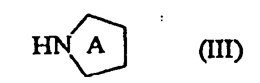 in der 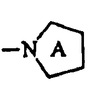 die bezüglich der Formel (I) angegebenen Bedeutungen besitzt, unter den klassischen Bedingungen der Peptidkupplung umsetzt,
so daß man nach der eventuellen Abspaltung der Schutzgruppe der Gruppe Y die Verbindung der Formel (IV) erhält: 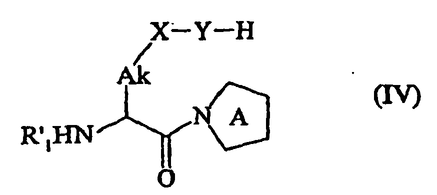 in der 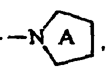 Ak, X, Y und R'₁ die oben angegebenen Bedeutungen besitzen, welche man anschließend mit einer Verbindung der Formel (V):
R₂ - SO₂ - Z₁ (V)
in der R₂ die bezüglich der Formel (I) angegebenen Bedeutungen besitzt und Z₁ eine austretende Gruppe, wie beispielsweise ein Halogenatom, darstellt, umsetzt,
so daß man nach der eventuellen Abspaltung der Schutzgruppe die Verbindung der Formel (I) erhält,
die man gegebenenfalls mit Hilfe einer klassischen Reinigungsmethode reinigt, gewünschtenfalls mit Hilfe einer klassischen Trennmethode in ihre optischen Isomeren auftrennt und gewünschtenfalls in ihre Additionssalze mit einer pharmazeutisch annehmbaren Säure überführt.

7. Pharmazeutische Zubereitung, enthaltend als Wirkstoff eine Verbindung nach einem der Ansprüche 1 bis 5 in Kombination mit einem oder mehreren inerten, nichttoxischen und pharmazeutisch annehmbaren Trägermaterialien.

8. Pharmazeutische Zubereitung nach Anspruch 7 geeignet als Arzneimittel zur Inhibierung der Dipeptidyl-peptidase IV, zur Behandlung der Glucoseunverträglichkeit, von Erkrankungen, die mit einer Hyperglykämie verknüpft sind, wie Diabetes Typ II oder Fettsucht, bei der Vorbeugung der Abstoßung von Transplantaten, bei der Behandlung von Krebs und zur Vorbeugung von Krebsmetastasen, bei der Behandlung von AIDS, der Parodontitis sowie bei der Darmadaptation nach Resektion.

9. Pharmazeutische Zubereitung nach Anspruch 7, geeignet als antidiabetisches Arzneimittel.

## Claims

1. Compound of formula (I) : 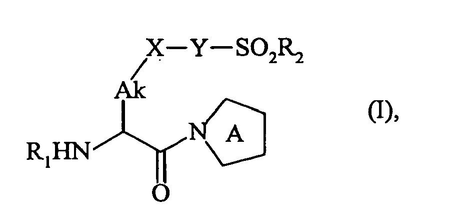
wherein:
- 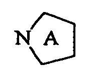 represents a 5-membered, nitrogen-containing heterocycle optionally substituted by a cyano group,
- R₁ represents a hydrogen atom or a linear or branched (C₁-C₆)alkyl, linear or branched (C₁-C₆)acyl, prolyl, alanyl, histidylprolyl or phenylalanylprolyl group,
- Ak represents a linear or branched (C₁-C₆)alkylene chain or a heteroalkylene chain,
- X represents a single bond or a phenylene group optionally substituted by one or more identical or different groups selected from halogen atoms and linear or branched (C₁-C₆)alkyl, hydroxy, linear or branched (C₁-C₆)alkoxy and linear or branched (C₁-C₆)polyhaloalkyl groups,
- R₂ represents a (C₃-C₁₀)cycloalkyl group,
- Y represents an NH group,
its optical isomers and addition salts thereof with a pharmaceutically acceptable acid,
a 5-membered, nitrogen-containing heterocycle being understood to mean a saturated, monocyclic, 5-membered group containing one, two or three hetero atoms, one of those hetero atoms being the nitrogen atom and the additional hetero atom(s) optionally present being selected from oxygen, nitrogen and sulphur atoms,
and a heteroalkylene chain being understood to mean a linear or branched (C₁-C₆)alkylene chain wherein a -CH₂- member has been replaced by an oxygen or sulphur atom.

2. Compound of formula (I) according to claim 1, wherein 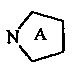 represents a 1-pyrrolidinyl group optionally substituted by a cyano group, or a 1,3-thiazolidin-3-yl group optionally substituted by a cyano group.

3. Compound of formula (I) according to claim 1 or 2, wherein X represents a single bond.

4. Compound of formula (I) according to any one of claims 1 to 3, wherein the configuration at the position α to the amide function 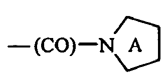 is (S).

5. Compound of formula (I) according to claim 1, which is (S)-N-[4-amino-5-oxo-5-(1-pyrrolidinyl)-pentyl]-cyclohexanesulphonamide, its optical isomers and also addition salts thereof with a pharmaceutically acceptable acid.

6. Process for the preparation of compounds of formula (I) according to claim 1, **characterised in that** a compound of formula (II) : 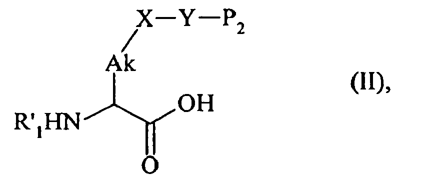 wherein Ak, X and Y are as defined for formula (I), R'₁ represents a protecting group for the amino function, or a linear or branched (C₁-C₆)alkyl group, a linear or branched (C₁-C₆)acyl group, a prolyl group optionally protected by an amino-function-protecting group, an alanyl group optionally protected by an amino-function-protecting group, a histidylprolyl group optionally protected by an amino-function-protecting group, or a phenylalanylprolyl group optionally protected by an amino-function-protecting group, and P₂ represents a hydrogen atom or a protecting group for the amino function,
is reacted with a compound of formula (III) : 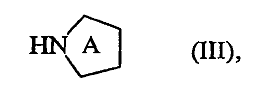 wherein 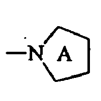 is as defined for formula (I),
under conventional conditions of peptide coupling,
to yield, after deprotection - where necessary - of the group Y, the compound of formula (IV) : 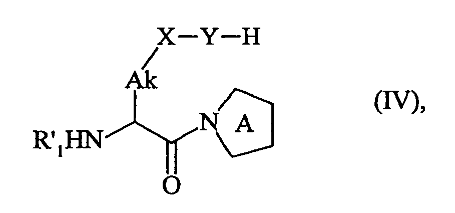 wherein 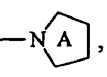 Ak, X, Y and R'₁ are as defined hereinbefore,
which is then reacted with a compound of formula (V) :
R₂-SO₂-Z₁ (V),
wherein R₂ is as defined for formula (I) and Z₁ represents a leaving group such as, for example, a halogen atom,
to yield, after deprotection where necessary, the compound of formula (I),
which is purified, where appropriate, according to a conventional purification technique, which is separated, if desired, into its optical isomers according to a conventional separation technique, and which is converted, if desired, into addition salts thereof with a pharmaceutically acceptable acid.

7. Pharmaceutical composition comprising as active ingredient a compound according to any one of claims 1 to 5, in combination with one or more inert, non-toxic and pharmaceutically acceptable vehicles.

8. Pharmaceutical composition according to claim 7 for use as a medicament as an inhibitor of dipeptidyl-peptidase IV, for use in the treatment of glucose intolerance and of disorders associated with hyperglycaemia such as type II diabetes or obesity, in the prevention of transplant rejection after transplantation, in the treatment of cancer and the prevention of cancerous metastases, in the treatment of AIDS and of periodontitis, and also in recovery of the intestine after resection.

9. Pharmaceutical composition according to claim 7 for use as an antidiabetic medicament.
